# EUROPEAN PATENT APPLICATION

(11) **EP 4 378 486 A1**
(43) Date of publication of application: **05.06.2024**
(21) Application number: 22383171.0
(22) Date of filing: 02.12.2022
(51) Int. Cl.: A61K 48/00, A61K 38/47

(54) **SECRETED SPLICING VARIANT OF KLOTHO FOR EXTENDING LIFESPAN**

(71) Applicant: Universitat Autònoma de Barcelona, 08193 Bellaterra (Cerdanyola del Valles) (ES); Institució Catalana de Recerca I Estudis Avançats, 08010 Barcelona (ES); Fundació Hospital Universitari Vall d'Hebron - Institut de Recerca, 08035 Barcelona (ES)
(72) Inventor: CHILLON RODRIGUEZ, Miguel, 08195 SANT CUGAT DEL VALLÈS (ES); BOSCH MERINO, Assumpció, 08195 SANT CUGAT DEL VALLÈS (ES); ROIG SORIANO, Joan, 12004 CASTELLÓ DE LA PLANA (ES)
(74) Representative: ZBM Patents - Zea, Barlocci & Markvardsen

(57) **Abstract**

The present invention provides a polypeptide consisting of sequence SEQ ID NO: 1, or a variant thereof consisting of a sequence at least 85% identical to SEQ ID NO: 1, for use in extending the lifespan of a subject. The invention also provides nucleic acid sequence that encodes the polypeptide, a gene construct comprising the nucleic acid sequence, or an expression vector comprising the gene construct, for said use. The polypeptide, nucleic acid sequence, gene construct, or expression vector of the invention may be administered in the form of a pharmaceutical composition together with at least one pharmaceutically acceptable excipient, diluent or carrier.

## Description

### Technical Field

The present invention relates to approaches for extending the lifespan of organisms.

### Background Art

Chronological age is well understood to be the single greatest risk factor for nearly every major cause of mortality and morbidity in living organisms, including humans. Even before the development of observable disease, the physiology of organ systems and tissues progressively declines throughout life. Thus, interventions to extend the lifespan -i.e., to slow the progression of aging *per se-,* could delay the onset of age-related diseases and death collectively, which would be a much more efficient therapeutic approach than treating age-related diseases individually.

Although our understanding of the biology of aging and longevity has grown tremendously over the past decades, it has been proved difficult to identify lifespan extending treatments and to elucidate the molecular mechanisms underlying longevity. Thus, products and methods that extend lifespan have generally been proven to be ineffective and/or unsafe.

Most of studies that have reported lifespan prolongation in animals have been carried out in animal models with specific diseases or conditions. As a consequence, most of the time the interventions are thought not to extend lifespan *per se,* but rather as a consequence of preventing or treating the specific condition. Thus, identifying treatments with lifespan extending effects *per se* -in contrast to disease treatments that indirectly extend lifespan in diseased animals- has proved difficult.

The Klotho gene was discovered on 1997 as a gene whose absence in mice conferred an accelerated aging, or progeroid, phenotype with a dramatically shortened life span. Yet, despite intensive research over the last 25 years since Klotho's discovery, there are currently no available treatments or clinical trials based on Klotho for lifespan extension.

Thus, in spite the efforts made so far, there remains a need in the art for safe compounds with the capacity to extend the lifespan of a subject.

### Summary of Invention

The present inventors have developed a novel therapy for the extension of lifespan based on the administration of secreted splicing isoform of Klotho, s-KL.

The Klotho gene presents two main transcripts -full-length Klotho mRNA and alternatively spliced Klotho mRNA. Full-length Klotho mRNA transcribes a single-pass transmembrane protein called m-KL, of 135KDa. The extracellular domains of m-KL can be released from the membrane by protease-mediated shedding, generating soluble, circulating processed Klotho (p-KL, of 130KDa) which is sometimes simply called soluble Klotho, and has two active domains. The alternatively spliced Klotho mRNA shows a premature stop codon and generates a secreted protein, s-KL (70KDa), containing just one of the active domains and an extra 15 amino acids at its C-terminus. Although a similar abbreviation (s-KL) is sometimes used in the prior art to name soluble Klotho - which is the processed version of transmembrane full-length Klotho- and secreted Klotho -which is the splicing variant-, these two variants present a completely different structure, size, and biological activities. In fact, while full-length Klotho has been described to be involved in FGF23 receptor binding, PTH synthesis, regulation of parathyroid gland growth, and alterations in Vitamin D metabolism and Calcium ion blood levels, the secreted splicing variant of Klotho (s-KL) has not.

The present invention is exclusively based on the use of the splicing variant, secreted Klotho, whose biological functions remain largely unknown, and the abbreviation s-KL is used herein to refer to secreted Klotho only.

Surprisingly, the present inventors found that s-KL treatment increased both median and total lifespan in mice, both when administered in a young developmental stage (i.e., 6 months) and in an adult developmental stage (i.e., 12 months) without causing any adverse effects.

It must be noted that the results herein provided were obtained in a naive (i.e., non-diseased) animal model, therefore they allow to draw conclusions regarding the positive direct effects of s-KL on the lifespan of animals -and rule out that the results are an indirect consequence of disease treatment. Also, the results were obtained by administering exogenous s-K, and not by genetic manipulation of the animals, which clearly indicate that the anti-aging effect of s-KL proposed herein can be readily translated into clinical applications to promote longevity.

In view of the above, the present invention constitutes a great advance in the field of lifespan extension therapies and may help prevent all kind of age-related diseases.

Thus, in a first aspect, the present invention provides a polypeptide consisting of sequence SEQ ID NO: 1, or a variant thereof consisting of a sequence at least 85% identical to SEQ ID NO: 1, for use in extending the lifespan of a subject.

In a second aspect, the present invention provides a nucleic acid sequence that encodes the polypeptide or the variant thereof as defined in the first aspect, which is for use in extending the lifespan of a subject.

In a third aspect, the present invention provides a gene construct comprising a nucleic acid sequence as defined in the second aspect, operatively linked to an expression promoter, which is for use in extending the lifespan of a subject.

In a fourth aspect, the present invention provides and expression vector comprising the gene construct as defined in the third aspect, which is for use in extending the lifespan of a subject.

In a fifth aspect, the invention provides a host cell which is transformed or transfected with the nucleic acid sequence as defined in the second aspect, the gene construct as defined in the third aspect, or the expression vector as defined in the fourth aspect, which is for use in extending the lifespan of a subject.

In a sixth aspect, the invention provides a non-therapeutic method for extending the lifespan of a subject, the method comprising administering to the subject a polypeptide consisting of sequence SEQ ID NO: 1 or a variant thereof consisting of a sequence at least 85% identical to SEQ ID NO: 1, or a nucleic acid sequence that encodes the polypeptide or the variant thereof.

In a seventh aspect, the invention provides the use of a polypeptide consisting of sequence SEQ ID NO: 1 or a variant thereof consisting of a sequence at least 85% identical to SEQ ID NO: 1; or a nucleic acid sequence encoding the polypeptide or the variant thereof, for extending the lifespan of a subject.

### Brief Description of Drawings

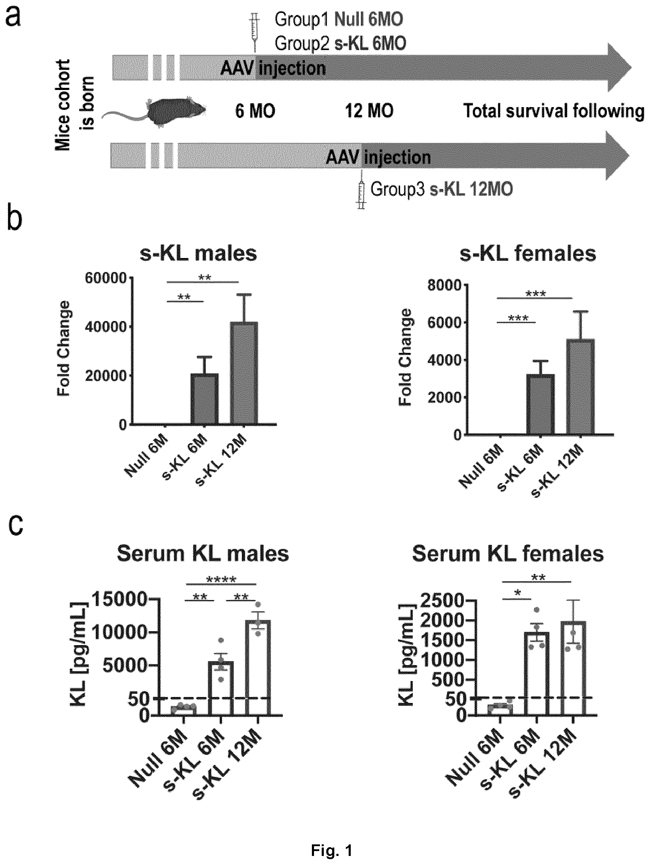

Fig. 1. s-KL treatment efficiently increased s-KL protein concentration. a) Schematic representation of the experimental design. b) s-KL gene expression analysis in liver of males (left panel) and females (right panel). c) Quantification of total s-KL protein concentration in serum of males (left panel) and females (right panel). Analysis done with samples of a subset of animals euthanized at the age of 24 months old. Data represented in (b) as fold change expression respect Null treated animals. Mean ± Standard error of the mean (SEM), n=4; *p<0.05; **p<0.01; ***p<0.001; ****p<0.001.

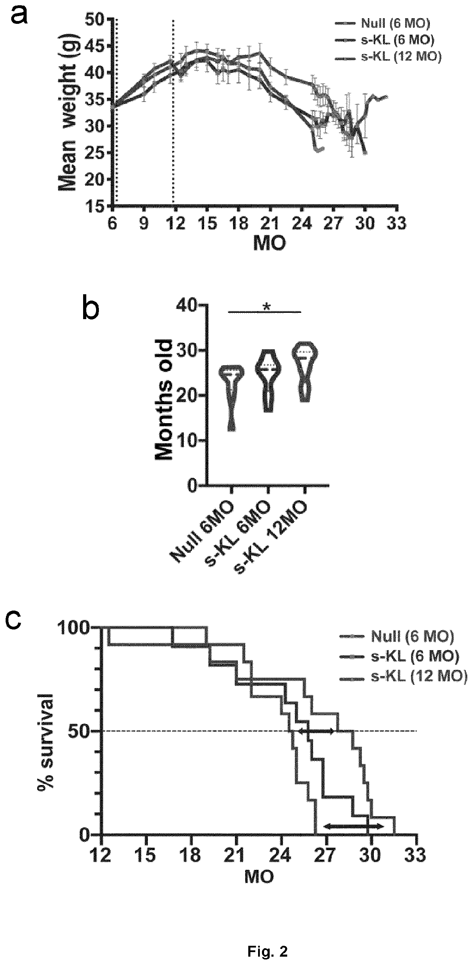

Fig. 2. s-KL treatment increases median and total longevity in wild-type mice. a) Body weight follow up. b) Violin plots of the median survival of the different treatments studied. Mean ± Standard error of the mean (SEM), n=11-12; *p<0.05. c) Longevity evolution of the different groups represented with Kaplan-Meyer longevity curves.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

As used herein, the indefinite articles "a" and "an" are synonymous with "at least one" or "one or more." Unless indicated otherwise, definite articles used herein, such as "the" also include the plural of the noun.

As above discussed, in a first aspect, the invention provides a polypeptide consisting of sequence SEQ ID NO: 1, or a variant thereof consisting of a sequence at least 85% identical to SEQ ID NO: 1, for use in extending the lifespan of a subject.

This aspect can also be formulated as the use of a polypeptide as defined above for the manufacture of a medicament for extending the lifespan of a subject. The present invention also relates to a method for extending the lifespan of a subject, comprising administering a therapeutically effective amount of a polypeptide as defined above, together with pharmaceutically acceptable excipients or carriers, to the subject.

In a more particular embodiment of the first aspect of the invention, the polypeptide consists of sequence SEQ ID NO: 1 or a variant thereof consisting of a sequence at least 85%, 86%, 87%, 88%, 88.5%, 89%, 89.5%, 90%, 90.5%, 91%, 91.5%, 92%, 92.5%, 93%, 93.5%, 94%, 94.5%, 95%, 95.5%, 96%, 96.5%, 97%, 97.5%, 98%, 98.5%, 99%, or 99.5% identical to SEQ ID NO: 1. In an even more particular embodiment, the polypeptide consists of sequence SEQ ID NO: 1 or a variant thereof consisting of a sequence at least 88% or 98% identical to SEQ ID NO:1.

In a more particular embodiment of the first aspect of the invention, the polypeptide consists of sequence SEQ ID NO: 1 or a variant thereof consisting of a sequence at least 85%, 86%, 87%, 88%, 88.5%, 89%, 89.5%, 90%, 90.5%, 91%, 91.5%, 92%, 92.5%, 93%, 93.5%, 94%, 94.5%, 95%, 95.5%, 96%, 96.5%, 97%, 97.5%, 98%, 98.5%, 99%, or 99.5% identical to SEQ ID NO: 1, wherein the variant thereof substantially maintains or improves the lifespan extending effect of SEQ ID NO: 1.

In another embodiment of the first aspect of the invention, the polypeptide consists of sequence SEQ ID NO: 1 or SEQ ID NO: 2.

Protein variants are well understood to those of skill in the art and can involve amino acid sequence modifications. For example, amino acid sequence modifications typically fall into one or more of three classes: substitutional, insertional, or deletional variants.

In the present invention the term "identity" refers to the percentage of residues that are identical in the two sequences when the sequences are optimally aligned. If, in the optimal alignment, a position in a first sequence is occupied by the same amino acid residue as the corresponding position in the second sequence, the sequences exhibit identity with respect to that position. The percentage of identity determines the number of identical residues over a defined length in a given alignment. Thus, the level of identity between two sequences or ("percent sequence identity") is measured as a ratio of the number of identical positions shared by the sequences with respect to the number of positions compared (i.e., percent sequence identity = (number of identical positions/total number of positions compared) × 100). A gap, i.e., a position in an alignment where a residue is present in one sequence but not in the other, is regarded as a position with non-identical residues and is counted as a compared position.

As an illustration, by a polypeptide having an amino acid sequence having at least, for example, 95% identity to a reference amino acid sequence of SEQ ID NO:1 is intended that the amino acid sequence of the polypeptide is identical to the reference sequence except that the polypeptide sequence may include up to five amino acid alterations per each 100 amino acids of the reference amino acid of SEQ ID NO: 1. In other words, to obtain a polypeptide having an amino acid sequence of at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

A number of mathematical algorithms for rapidly obtaining the optimal alignment and calculating identity between two or more sequences are known and incorporated into a number of available software programs. For purposes of the present invention, the sequence identity between two amino acid sequences is preferably determined using algorithms based on global alignment, such as the Needleman-Wunsch algorithm (Needleman and Wunsch, 1970, J. Mol. Biol. 48: 443-453), preferably implemented in the Needle program of the EMBOSS package (EMBOSS: The European Molecular Biology Open Software Suite, Rice et al., 2000, Trends Genet. 16: 276-277); or the BLAST Global Alignment tool (Altschul et al., "Basic local alignment search tool", 1990, J. Mol. Biol, v. 215, pages 403-410), using default settings. Local alignment also can be used when the sequences being compared are substantially of the same length.

The polypeptides with a percentage of identity of at least 88 % with any of SEQ ID NO: 1 or SEQ ID NO: 2 encompass s-KL of mammals other than mice and human.

SEQ ID NO: 1 is the amino acid sequence of the transcript from alternative splicing of α-klotho human gene, comprising the KL1 domain sequence, with an approximate weight of 70 kDa and a specific secretion signal consisting of 15 amino acid tail that is not found in the m-KL transcript. α-klotho human gene is the one located in Chromosome 13 NC_000013.11 (33016063..33066145) of the assembly GRCh38 (24.12.2013) for the human genome maintained by the Genome Reference Consortium. SEQ ID NO: 1 derives from the corresponding cDNA of SEQ ID NO: 3, deriving from the alternative splicing transcript of the mRNA sequence with the GenBank database accession number NM_004795 of 5012 base pairs, version 3 of 03.May.2014.

SEQ ID NO: 2 is the amino acid sequence of the transcript from alternative splicing of α-klotho mouse gene, comprising the KL1 domain sequence, with an approximate weight of 70 kDa with a specific secretion signal consisting of 15 amino acid tail that is not found in the m-KL transcript. α-klotho mouse gene is the one located in Chromosome 5 (150,952,607-150,993,809) of UCSC Genome Browser on Mouse July 2007 (NCBI37/mm9) Assembly for the mouse genome. SEQ ID NO: 2 derived from the corresponding cDNA of SEQ ID NO: 4, deriving in turn from the alternative splicing transcript of the mRNA sequence with the GenBank database accession number NM_013823 of 5124 base pairs, version 2 of 15.February.2015.

In another embodiment of the first aspect of the invention, the polypeptide variant consists of sequence SEQ ID NO: 5 or SED ID NO: 6.

In another embodiment of the first aspect of the invention, the polypeptide has a length equal to or lower than 645 amino acids, 600 amino acids, or 550 amino acids. In an even more particular embodiment, the polypeptide consists of sequence SEQ ID NO: 1 or a variant thereof consisting of a sequence at least 85% identical to SEQ ID NO: 1 and has a length equal to or lower than 645 amino acids, 600 amino acids, or 550 amino acids. In a particular embodiment, the polypeptide consists of sequence SEQ ID NO: 1 or a variant thereof consisting of a sequence at least 85% identical to SEQ ID NO: 1, and said variant has a length selected from the group consisting of 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 584, 586, 587, 588, 589, 590, 591, 592, 593, 594, 595, 596, 597, 598, 599, and 600 amino acids, or a length from 545 to 600 amino acids.

In one embodiment of the first aspect of the invention, the polypeptide is the secreted splicing variant of mammalian klotho protein (s-KL). In an even more particular embodiment, the polypeptide is the human s-KL. The secreted splicing variant of mammalian Klotho protein (s-KL) has been disclosed in the prior art (see, for example, WO2017085317A1). Thus, the invention can be in particular formulated as secreted splicing variant of mammalian Klotho protein (s-KL), in particular human s-KL, or nucleic acid sequence coding therefor, for use in extending the lifespan of a subject.

The term "secreted splicing variant of mammalian Klotho" abbreviated as "s-KL", refers to the protein resulting from the transcript from alternative splicing, which generates a truncated form of the protein (s-KL) that is formed by the KL1 domain, with an approximate weight of 70 kDa, together with a specific secretion signal consisting of 15 amino acid tail that is not found in the m-KL transcript, and for this reason is also called the secreted isoform of klotho, s-KL, or the secreted splicing variant of klotho protein. s-KL is different from other forms of soluble klotho, namely p-KL, p-KL1 and p-KL2. In this description, m-KL stands for the full-length transmembrane form; p-KL stands for the soluble proteolyzed klotho, which is generated by cleavage of the m-KL; and p-KL1 and p-KL2 stand for the soluble klotho forms consisting on the KL1 domain and the KL2 domain of p-KL, respectively. m-KL comes from the full-length transcript encoding a single pass transmembrane protein with a molecular weight of approximately 130 kDa (m-KL). The protein contains three domains: a short transmembrane domain at the C-terminal, an extracellular domain composed of two internal repeated sequences of about 550 amino acids called KL1 and KL2 respectively, and a very short intracellular domain of 10 amino acids. The extracellular domain of the transmembrane form can be cleaved by metalloproteinases ADAM10 and ADAM17 resulting in another form of soluble Klotho of about 130 kDa (abbreviated p-KL for proteolyzed membrane isoform. Moreover, there is a second recognition site for the proteases ADAM 10 and 17 located between the KL1 and KL2 domains, which generates two new 70 kDa isoforms, one contained the KL1 domain only (like the one generated from alternative splicing but without the specific amino acid tail), and the other one contained the KL2 domain. However, it has not been demonstrated *in vivo* that p-KL is proteolyzed into p-KL1 and p-KL2.

As used herein, "extending the lifespan" refers to increasing the average or maximum length of time an organism can be expected to survive or last. In particular, it refers to increasing the maximum lifespan. "Maximum lifespan" refers to the age at which the oldest known member of the species has died. The present invention is particularly directed to increasing the maximum lifespan. The terms " extending the lifespan ", "prolonging the lifespan" and " promoting longevity" are used in the present invention interchangeably.

In a particular embodiment of the first aspect, optionally in combination with any of the embodiments provided above and below, the polypeptide is linked to a heterologous moiety.

As used herein, "heterologous moiety" refers to any molecule coupled to the polypeptide via either a covalent or non-covalent bond. In a particular embodiment, the heterologous moiety is located in either the N-terminal or the C-terminal end of the polypeptide. In a particular embodiment, the heterologous moiety is located in both the N-terminal and the C-terminal ends of the polypeptide.

The heterologous moiety can be, for example, a molecule that facilitates the purification of the polypeptide. In a particular embodiment, the heterologous moiety is a peptide. In an even more particular embodiment, the heterologous moiety is a poly histidine track. As the skill in the art would understand, small peptides that assist in the purification of the protein can be maintained in the final compound without affecting its functionality.

The heterologous moiety can also be any vehiculization agent to facilitate the absorption, transport and delivery of the polypeptide.

These polypeptides resulting from KL protein, such as in particular s-KL, may be used directly in the form of the protein, or they can be expressed inside target cells of the tissue of interest by means of gene therapy. To this aim the invention also provides, in a second aspect, a nucleic acid sequence that encodes the polypeptide or the variant thereof as defined in the first aspect, which is for use in extending the lifespan of a subject.

The term "a nucleic acid sequence that encodes the polypeptide" is to be understood, in particular, as the mRNA coding for said polypeptide or the cDNA sequence resulting from the reverse transcription (RT-PCR) of the mRNA coding for said polypeptide.

This aspect can also be formulated as the use of the nucleic acid sequence as defined above for the manufacture of a medicament for extending the lifespan of a subject. The present invention also relates to a method for extending the lifespan of a subject, comprising administering a therapeutically effective amount of the nucleic acid sequence as defined above, together with pharmaceutically acceptable excipients or carriers, to the subject.

In a particular embodiment of the second aspect, the nucleic acid sequence comprises SEQ ID NO: 3 or SEQ ID NO: 4. In an even more particular embodiment, the nucleic acid sequence consists of SEQ ID NO: 3 or SEQ ID NO: 4.

In a more particular embodiment of the second aspect of the invention, the nucleic acid sequence consists of sequence SEQ ID NO: 3 or SEQ ID NO: 4 or a variant thereof consisting of a sequence at least 85%, 86%, 87%, 88%, 88.5%, 89%, 89.5%, 90%, 90.5%, 91%, 91.5%, 92%, 92.5%, 93%, 93.5%, 94%, 94.5%, 95%, 95.5%, 96%, 96.5%, 97%, 97.5%, 98%, 98.5%, 99%, or 99.5% identical to SEQ ID NO: 3 or SEQ ID NO: 4.

In a more particular embodiment of the second aspect of the invention, the nucleic acid sequence consists of sequence SEQ ID NO: 3 or SEQ ID NO: 4 or a variant thereof consisting of a sequence at least 85%, 86%, 87%, 88%, 88.5%, 89%, 89.5%, 90%, 90.5%, 91%, 91.5%, 92%, 92.5%, 93%, 93.5%, 94%, 94.5%, 95%, 95.5%, 96%, 96.5%, 97%, 97.5%, 98%, 98.5%, 99%, or 99.5% identical to SEQ ID NO: 3 or SEQ ID NO: 4, wherein the variant thereof substantially maintains or improves the lifespan extending effect of SEQ ID NO: 3 or SEQ ID NO: 4.

In a third aspect, the invention provides a gene construct comprising a nucleic acid sequence as defined in the second aspect operatively linked to an expression promoter, which is for use in extending the lifespan of a subject.

This aspect can also be formulated as the use of a gene construct as defined above for the manufacture of a medicament for extending the lifespan of a subject. The present invention also relates to a method for extending the lifespan of a subject, comprising administering to the subject a therapeutically effective amount of a gene construct as defined above, together with pharmaceutically acceptable excipients or carriers.

In a particular embodiment of the third aspect, the expression promoter operatively linked is selected from the group consisting of a constitutive expression promoter, an inducible promoter, a muscle-specific expression promoter, and a neuron-specific expression promoter. In a more particular embodiment, the gene construct according to the invention comprises the cytomegalovirus intermediate-early (CMV IE) promoter, the sequence coding for s-KL (cDNA of mouse or human s-KL) and a polyadenylation chain (poly A). In another particular embodiment, the gene construct according to the invention comprises the CAG promoter, the sequence coding for s-KL (cDNA of mouse or human s-KL) and a polyadenylation chain (poly A).

In a particular embodiment of the third aspect, optionally in combination with any of the embodiments provided above and below, the gene construct comprises or consists of SEQ ID NO: 7 or SEQ ID NO: 8.

All these gene constructs are able to express the protein of interest once in the cell.
In order to facilitate administration of the constructs, the invention also provides, in a fourth aspect, an expression vector comprising the gene construct as defined in the third aspect and thus comprising the nucleic acid sequence of the second aspect coding for the polypeptide of the first aspect operatively linked to an expression promoter, and particularly to a constitutive expression promoter, for use in extending the lifespan of a subject.

This aspect can also be formulated as the use of the expression vector as defined above for the manufacture of a medicament for extending the lifespan of a subject. The present invention also relates to a method for extending the lifespan of a subject, comprising administering to the subject a therapeutically effective amount of the expression vector as defined above, together with pharmaceutically acceptable excipients or carriers.

In a particular embodiment of the fourth aspect, optionally in combination with any of the embodiments provided above and below, the expression vector is a viral vector.

In a particular embodiment of the third aspect, optionally in combination with any of the embodiments provided above and below, the expression vector consists of sequence SEQ ID NO: 9.

In a particular embodiment of the fourth aspect, optionally in combination with any of the embodiments provided above and below, the viral vector is an adeno-associated virus. In a particular embodiment, it an adeno-associated virus of serotype selected from the group consisting of AAV1, AAV2, AAV4, AAV5, AAV6, AAV7, AAV8, AAV9, AAVrh10, PHPeB, , and 9P31, which has the capacity to cross the BBB. In a more particular embodiment, it an adeno-associated virus of serotype AAV9.

In another embodiment of the first, second, third and fourth aspects, optionally in combination with any of the embodiments provided above and below, the polypeptide for use according to the first aspect, the nucleic acid sequence for use according to the second aspect, the gene construct for use according to the third aspect, or the expression vector for use according to the fourth aspect, is administered in the form of a pharmaceutical composition together with at least one pharmaceutically acceptable excipient, diluent or carrier.

The expression "pharmaceutical composition" encompasses both compositions intended for human as well as for non-human animals. The skilled in the art understands that a pharmaceutical composition must comprise a therapeutically effective amount of the compound. The expression "therapeutically effective amount" as used herein, refers to the amount of polypeptide, nucleic acid sequence, gene construct, or expression vector that, when administered, is sufficient to prevent development of, or alleviate to some extent, one or more of the symptoms of the disease which is addressed. The particular dose of compound administered according to this invention will of course be determined by the particular circumstances surrounding the case, including the compound administered, the route of administration, the particular condition being treated, and the similar considerations.

The expression "pharmaceutically acceptable excipient, diluent or carrier" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and non-human animals without excessive toxicity, irritation, allergic response, immunogenicity or other problems or complications commensurate with a reasonable benefit/risk ratio.

Examples of suitable pharmaceutically acceptable excipients are solvents, dispersion media, diluents, or other liquid vehicles, dispersion or suspension aids, surface active agents, isotonic agents, thickening or emulsifying agents, preservatives, solid binders, lubricants and the like. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this invention.

The relative amounts of the active ingredient, the pharmaceutically acceptable excipient, and/or any additional ingredients in a pharmaceutical composition of the invention will vary, depending upon the identity, size, and/or condition of the subject treated and further depending upon the route by which the composition is to be administered.

Pharmaceutically acceptable excipients used in the manufacture of pharmaceutical compositions include, but are not limited to, inert diluents, dispersing and/or granulating agents, surface active agents and/or emulsifiers, disintegrating agents, binding agents, preservatives, buffering agents, lubricating agents, and/or oils. Excipients such as coloring agents, coating agents, sweetening, and flavoring agents can be present in the composition, according to the judgment of the formulator.

The pharmaceutical compositions containing the protein or nucleic acid of the invention can be presented in any dosage form, for example, solid or liquid, and can be administered by any suitable route, for example, oral, parenteral, rectal, topical, intranasal, intraocular, intraperitoneal or sublingual route, for which they will include the pharmaceutically acceptable excipients necessary for the formulation of the desired dosage form, for example, topical formulations (ointment, creams, lipogel, hydrogel, etc.), eye drops, aerosol sprays, injectable hydrogels, injectable solutions, osmotic pumps, etc.

Exemplary diluents include, but are not limited to, calcium carbonate, sodium carbonate, calcium phosphate, dicalcium phosphate, calcium sulfate, calcium hydrogen phosphate, sodium phosphate lactose, sucrose, cellulose, microcrystalline cellulose, kaolin, mannitol, sorbitol, inositol, sodium chloride, dry starch, corn-starch, powdered sugar, and combinations thereof.

Exemplary granulating and/or dispersing agents include, but are not limited to, potato starch, corn starch, tapioca starch, sodium starch glycolate, clays, alginic acid, guar gum, citrus pulp, agar, bentonite, cellulose and wood products, natural sponge, cation-exchange resins, calcium carbonate, silicates, sodium carbonate, cross-linked polyvinylpyrrolidone) (crospovidone), sodium carboxymethyl starch (sodium starch glycolate), carboxymethyl cellulose, cross-linked sodium carboxymethyl cellulose (croscarmellose), methylcellulose, pregelatinized starch (starch 1500), microcrystalline starch, water insoluble starch, calcium carboxymethyl cellulose, magnesium aluminum silicate (Veegum), sodium lauryl sulfate, quaternary ammonium compounds, and combinations thereof.

Exemplary binding agents include, but are not limited to, starch (e.g., corn-starch and starch paste); gelatin; sugars (e.g., sucrose, glucose, dextrose, dextrin, molasses, lactose, lactitol, mannitol); natural and synthetic gums (e.g., acacia, sodium alginate, extract of Irish moss, panwar gum, ghatti gum, mucilage of isapol husks, carboxymethylcellulose, methylcellulose, ethylcellulose, hydroxyethylcellulose, hydroxypropyl cellulose, hydroxypropyl methylcellulose, microcrystalline cellulose, cellulose acetate, polyvinylpyrrolidone), magnesium aluminium silicate (Veegum), and larch arabogalactan); alginates; polyethylene oxide; polyethylene glycol; inorganic calcium salts; silicic acid; polymethacrylates; waxes; water; alcohol; and combinations thereof.

Exemplary preservatives may include antioxidants, chelating agents, antimicrobial preservatives, antifungal preservatives, alcohol preservatives, acidic preservatives, and other preservatives. Exemplary antioxidants include, but are not limited to, alpha tocopherol, ascorbic acid, ascorbyl palmitate, ascorbyl stearate, ascorbyl oleate, butylated hydroxyanisole, butylated hydroxytoluene, monothioglycerol, potassium metabisulfite, propionic acid, propyl gallate, sodium ascorbate, sodium bisulfite, sodium metabisulfite, and sodium sulfite. Exemplary chelating agents include ethylenediaminetetraacetic acid (EDTA), citric acid monohydrate, disodium edetate, dipotassium edetate, edetic acid, fumaric acid, malic acid, phosphoric acid, sodium edetate, tartaric acid, and trisodium edetate.

Exemplary buffering agents include, but are not limited to, citrate buffer solutions, acetate buffer solutions, phosphate buffer solutions, ammonium chloride, calcium carbonate, calcium chloride, calcium citrate, calcium glubionate, calcium gluceptate, calcium gluconate, D-gluconic acid, calcium glycerophosphate, calcium lactate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, magnesium hydroxide, aluminum hydroxide, alginic acid, pyrogen-free water, isotonic saline, Ringer's solution, ethyl alcohol, and combinations thereof.

Exemplary lubricating agents include, but are not limited to, magnesium stearate, calcium stearate, stearic acid, silica, talc, malt, glyceryl behanate, hydrogenated vegetable oils, polyethylene glycol, sodium benzoate, sodium acetate, sodium chloride, leucine, magnesium lauryl sulfate, sodium lauryl sulfate, and combinations thereof.

In another embodiment of the first, second, third and fourth aspects, optionally in combination with any of the embodiments provided above and below, the pharmaceutical composition is for being administered to the patient via mucosa (e.g., nasal, sublingual, vaginal, buccal, or rectal), parenterally (e.g., subcutaneous, intravenous, intramuscular, or intraarterial injection, either bolus or infusion), orally, transdermally or via inhalation by means e.g., of an aerosol. Formulations suitable for parenteral administration, such as, for example, by intraarticular, intravenous, intramuscular, intradermal, intraperitoneal, and subcutaneous routes, include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. Injection solutions and suspensions can also be prepared from sterile powders, granules, and tablets. In some embodiments, the composition is administered by injection e.g subcutaneous, intraperitoneal, intravesically, intravenous, intracerebroventricular, by infusion, e.g., using a reservoir or osmotic minipump or intramuscular. The formulation can be provided in unit-dose or multi-dose sealed containers, such as ampoules and vials. In an even more particular embodiment, the pharmaceutical composition is for intraventricular administration or for intravenous administration; even more particularly systemic intravenous administration.

In another embodiment of the first, second, third and fourth aspects, optionally in combination with any of the embodiments provided above and below, the polypeptide for use according to the first aspect, the nucleic acid sequence for use according to the second aspect, the gene construct for use according to the third aspect, or the expression vector for use according to the fourth aspect, is administered in combination with another active agent. Suitable active agents to be administered in combination with a compound of the invention are, without limitation, vitamin A, berberine, α-ketoglutarate, resveratrol, caffeine, 5-aminoimidazole-4-carboxamide-ip-d-ribonucleoside, 2- deoxyglucose, apigenin, metformin, quercetin, and rosaglitazone.

In an embodiment of the aspects described above, optionally in combination with any of the embodiments provided above or below, the subject is a healthy subject. In an embodiment of the aspects described above, optionally in combination with any of the embodiments provided above or below, the subject does not suffer an age-related disease. "Age-related disease" refers to disorders or diseases in which aging is an important risk factor.

In an embodiment of the aspects described above, optionally in combination with any of the embodiments provided above or below, the subject is a mammal. In a more particular embodiment, the mammal is dog, cat, horse, cow, pig, rabbit, sheep, rodent, non-human primate, or human. In an even more particular embodiment, the mammal is human.

In an embodiment of the aspects described above, optionally in combination with any of the embodiments provided above or below, the subject is selected from the group consisting of young subject, adult subject, and elderly subject. More particularly, the subject is human and is selected from the group consisting of young subject, adult subject, and elderly subject.

In an embodiment of the aspects described above, optionally in combination with any of the embodiments provided above or below, extending the lifespan comprises an increase of at least 5%, at least 10%, at least 15%, or at least 19%, in lifespan relative to the expected lifespan of a subject of similar species.

In another embodiment of the aspects above, the polypeptide, nucleic acid sequence, gene construct, or expression vector is administered for a particular time period or for a chronic treatment period, which is, for an extended period of time, including throughout the duration of the subject's life. Within the treatment period, the polypeptide, nucleic acid sequence, gene construct, or expression vector is administered on a particular time schedule. In further embodiments, the polypeptide, nucleic acid sequence, gene construct, or expression vector is administered one, two, three, or four times daily. In some embodiments, the polypeptide, nucleic acid sequence, gene construct, or expression vector is administered once daily. In some embodiments, the polypeptide, nucleic acid sequence, gene construct, or expression vector is administered twice daily. In some embodiments, the polypeptide, nucleic acid sequence, gene construct, or expression vector is administered in the morning and evening. In some embodiments, the polypeptide, nucleic acid sequence, gene construct, or expression vector is administered one, two, three, or four times weekly. In some embodiments, the polypeptide, nucleic acid sequence, gene construct, or expression vector is administered once a week. In some embodiments the polypeptide, nucleic acid sequence, gene construct, or expression vector is administered one, two, three, or four times monthly. In some embodiments, the polypeptide, nucleic acid sequence, gene construct, or expression vector is administered once a month. In some embodiments, the polypeptide, nucleic acid sequence, gene construct, or expression vector is administered for at least three months of every one year. In some embodiments, the polypeptide, nucleic acid sequence, gene construct, or expression vector is administered one month of every six months.

As above indicated, the invention provides in a sixth aspect a non-therapeutic method for extending the lifespan of a subject, the method comprising administering to the subject a polypeptide consisting of sequence SEQ ID NO: 1 or a variant thereof consisting of a sequence at least 85% identical to SEQ ID NO: 1, or a nucleic acid sequence that encodes the polypeptide or the variant thereof. The embodiments of the first and second aspect, particularly those related to the sequence of the polypeptide or variant thereof and the nucleic acid sequence, are also meant to apply to this sixth aspect.

In an embodiment of the sixth aspect of the invention, the nucleic acid sequence is comprised in a gene construct operatively linked to an expression promoter. In a more particular embodiment, the gene construct is comprised in an expression vector. The embodiments of the third, and fourth aspects related to expression construct, vector, and subject, are also meant to apply to the sixth aspect of the invention.

In an embodiment of the sixth aspect, the subject is a healthy subject. In another embodiment, the subject does not suffer an age-related disease. In another embodiment, the subject is selected from the group consisting of young subject, adult subject, and elderly subject. More particularly, the subject is human and is selected from the group consisting of young subject, adult subject, and elderly subject.

As mentioned above, the invention also provides in a seventh aspect the use of the polypeptide or the nucleic acid encoding it for extending the lifespan of a subject. All the embodiments of the aspects described above are also meant to apply to this seventh aspect.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim and shall not be construed as limiting the scope of the claim. Furthermore, the present invention covers all possible combinations of particular and preferred embodiments described herein.

### Examples

### Materials and methods

### Animal housing

C57BU6J male (n=48) and female mice (n=48) were purchased from Charles River. These animals were randomly divided into 3 groups per sex. Two of those were treated when they were 7 months old with a Null control vector (SEQ ID NO: 10) (n=16) or a s-KL expressing vector (SEQ ID NO: 9 (n=16). The third group was treated when animals were 12 months old with the s-KL expressing vector (SEQ ID NO: 9) (n=16). The main part of these animals was followed during the life span to study health and viability and a second subset of 4 animals per group were randomly selected and euthanatized when they were 24 months old, to study viral vector function.

Mice had free access to food and water and were kept under standard temperature conditions (22±2°C) and a 12-h light/dark cycle (300 lux/0 lux). Mice were periodically checked to address general health status until natural death or euthanasia. Euthanasia protocol was cervical dislocation, applied when a blinded veterinary at the animal house facility considered an animal had reached the end point criteria.

### Treatment generation and administration

Gene therapy treatment consisted in expression cassettes under the control of the CAG promoter containing a control-null sequence or the secreted isoform of mouse α-KL gene (SEQ ID NO: 2).

Two adeno-associated viral vectors serotype 9 (AAV9), containing independently those constructs, were generated following the triple transfection method as disclosed in Piedra J. X et al., 2015. Animals were administered simultaneous by intracerebroventricular and intravenous injection, to transduce most mice tissues as possible. Intracerebroventricular stereotaxic injections of AAV vectors were performed as previously described (Massó A. et al., "Secreted αKlotho isoform protects against age-dependent memory deficits" Mol Psychiatry, 2018, vol. 23(9), pp. 1937-1947). Briefly, the treatment was administered into the right hemisphere at coordinates, -0.2 mm antero-posterior, -2 mm dorso-ventral, and +1 mm medio-lateral from bregma. The vector dose was 1×10¹¹ viral genomes per animal in 6 µl, administered at a 0.5 µl/min speed using an ultramicropump (WorldPrecision Instruments). The intravenous injection consisted in a dose of 4×10¹¹ viral genomes per animal diluted with NaCl 0.9% to a final volume of 200 µL and injected manually with a syringe into the lateral tail vein of the mice.

### Serum biochemical analysis

Blood samples were obtained by decapitation of deeply anesthetized animals, with a SST serum collection tube (BD microtainer). Blood was left at room temperature for 5 minutes and then placed on ice. Blood serum was isolated by 15 minutes tube centrifugation at 3000 rpm for 10 minutes, and finally aliquoted and kept frozen at -80 °C. KL serum levels were measured using an ELISA kit specific for mouse KL (IBL) following manufacturers indications.

### Gene expression

Total RNA isolation was carried out using TRIsure^{™} reagent following the manufacturer's instructions (Bioline Reagent). The tissue used for the RNA extraction was liver. Samples were homogenized using TissueLyser LT sample disruption apparatus (QIAGEN). RNA quantity and purity were measured with NanoDrop^{™} 1000 Spectrophotometer (Thermo Scientific). RNA retrotranscription was done using iScript^{™} Advanced cDNA Synthesis Kit (Bio-rad). Gene expression was analyzed by Real-Time quantitative PCR (RT-qPCR) on a Bio-Rad CFX-384 PCR machine at the Analysis and Photodocumentation Service of the Universitat Autonòma de Barcelona following manufacturer's instructions. Each reaction contained 25 ng of cDNA, 7.5 µL of iTaqTM Universal SYBR Green Supermix (Bio-Rad) and a primer concentration of 0.2 nM, with a final reaction volume of 15 µL. Primers used are listed in Table 1.

**Table 1**

| **Target** | **Product size (bp)** | **Forward primer (5'-3')** | **Reverse primer (5'-3')** |
|---|---|---|---|
| ***s-KL*** | 315 | TCATAATGGAAACCTTAAAAGCA A (SEQ ID NO: 11) | CACTGGGTTTTGTCAAAG GA (SEQ ID NO: 12) |
| ***β-actin*** | 190 | CAACGAGCGGTTCCGAT (SEQ ID NO: 13) | GCCACAGGTTCCATACCC A (SEQ ID NO: 14) |

### Statistical analysis

Statistical analysis and graphic representation were done with GraphPad Prism ver.8 (GraphPad Software). Statistical differences between groups were analyzed with a two-tailed unpaired Student's t-test when comparing two groups, one-way analysis of variance (ANOVA), followed by Tukey as a post-hoc analysis for comparing all treatment groups. Longevity curves were represented as Kaplan-Meyer longevity curves and the statistical difference was analyzed using the Log-rank (Mantel-Cox) statistical test. Data were expressed as mean ± standard error of the mean (SEM). Statistical difference was accepted when p values were ≤0.05.

### Results

### s-KL treatment efficiently increased s-KL protein concentration

Viral vector administration was done by consecutive intravenous and intra cerebroventricular injection (fig. 1a). Out of the 96 animals treated, one died just after the intervention. At 24 months of age, a randomly selected subset of 4 animals of each group were euthanatized in order to assess viral vector function. Gene expression of s-KL was studied in liver because this organ is transfected after AAV9 serotype injection and is the main secreting organ in adult animals (fig. 1b). Expression of the s-KL cDNA was significantly increased in all KL-treated groups, being higher in males than in females, and in the 12MO (12 months old) group compared to the 6MO (6 months old) treated animals. Additionally, efficient protein production and secretion to bloodstream was confirmed by ELISA (fig. 1c). Significant higher KL protein levels in serum were detected in mice administered both at 6 and 12 months of age, compared to animals treated with the AAV9 containing the Null. Again, the concentration was much higher in males than females. In the case of the males, it was also significantly increased in 12MO males compared to the 6MO group, presenting more than double of the s-KL concentration.

### s-KL treatment extended the lifespan of treated mice

Body weight is an important variable for health status. In males, mean body weight picked at the age of 14 months for Null, 15 months s-KL 6MO and was delayed until 20 months in the s-KL 12MO group (fig. 2a).

As shown in figure 2b, s-KL treatments increased longevity (i.e., lifespan). Median survival was of 24.6, 25.8, and 28.3 months for Null treated, sKL-6MO and sKL-12MO respectively, which means a statistical difference of 15% of increment in life expectancy of s-KL 12MO respect to Null treated animals.

Finally, total longevity (i.e., maximum lifespan) of the animals was also monitored, and the results are showed in Kaplan-Meier survival curves (fig. 2c). Total longevity was of 26.3, 29.8 and 31.5 months for Null treated, s-KL 6MO and s-KL 12MO respectively, which means a 19,7% increment. The comparison of total survival curve of the groups presented p-values in the Long-rank Mantel-Cox test of 0.08 (Null 6MO compared to s-KL 6MO) and 0.005 (Null 6MO compared to s-KL 12MO).

All these results clearly indicate that s-KL administration may be a useful therapy for extending the lifespan of animals.

### Citation List

Altschul et al., "Basic local alignment search tool", 1990, J. Mol. Biol, vol. 215, pp. 403-410.
WO2017085317A1
Massó A. et al., "Secreted αKlotho isoform protects against age-dependent memory deficits" Mol Psychiatry, 2018, vol. 23(9), pp. 1937-1947
Piedra, J. et al., "Development of a rapid, robust, and universal picogreen-based method to titer adeno-associated vectors", 2015, Human Gene Therapy Methods, vol. 26(1), pp. 35-42

## Claims

1. Polypeptide consisting of sequence SEQ ID NO: 1, or a variant thereof consisting of a sequence at least 85% identical to SEQ ID NO: 1, for use in extending the lifespan of a subject.

2. The polypeptide for use according to claim 1, wherein the polypeptide consists of sequence SEQ ID NO: 1 or a variant thereof consisting of a sequence at least 88 % identical to SEQ ID NO: 1.

3. The polypeptide for use according to any of claims 1-2, wherein the polypeptide consists of sequence SEQ ID NO: 1 or a variant thereof consisting of a sequence at least 98 % identical to SEQ ID NO: 1.

4. The polypeptide for use according to any of claims 1-3, wherein the polypeptide consists of SEQ ID NO: 1 or SEQ ID NO: 2.

5. Nucleic acid sequence that encodes the polypeptide or the variant thereof as defined in any of claims 1-4, which is for use in extending the lifespan of a subject.

6. Gene construct comprising a nucleic acid sequence as defined in claim 5, operatively linked to an expression promoter, which is for use in extending the lifespan of a subject.

7. Expression vector comprising the gene construct as defined in claim 6, which is for use in extending the lifespan of a subject.

8. The expression vector for use according to claim 7, which is a viral vector.

9. The expression vector for use according to claim 8, which is an adeno-associated virus of serotype selected from the group consisting of AAV1, AAV2, AAV4, AAV5, AAV6, AAV7, AAV8, AAV9, AAVrh10, PHPeB, and 9P31.

10. The polypeptide for use according to any of claims 1-4, the nucleic acid sequence for use according to claim 5, the gene construct for use according to claim 6, or the expression vector for use according to any of claims 7-9, wherein the subject is a mammal, particularly a human.

11. The polypeptide for use according to any of claims 1-4 and 10, the nucleic acid sequence for use according to claim 5 and 10, the gene construct for use according to claim 6 and 10, or the expression vector for use according to any of claims 7-10, wherein the subject is a healthy subject.

12. The polypeptide for use according to any of claims 1-4 and 10-11, the nucleic acid sequence for use according to claim 5 and 10-11, the gene construct for use according to claim 6 and 10-11, or the expression vector for use according to any of claims 7-11, which is administered in the form of a pharmaceutical composition together with at least one pharmaceutically acceptable excipient, diluent or carrier.

13. The polypeptide for use according to any of claims 1-4 and 10-12, the nucleic acid sequence for use according to claim 5 and 10-12, the gene construct for use according to claim 6 and 10-12, or the expression vector for use according to any of claims 7-12, which is administered in combination with another active agent.

14. A non-therapeutic method for extending the lifespan of a subject, the method comprising administering to a subject a polypeptide consisting of sequence SEQ ID NO: 1 or a variant thereof consisting of a sequence at least 85% identical to SEQ ID NO: 1; or a nucleic acid sequence encoding the polypeptide or the variant thereof.

15. Use of a polypeptide consisting of sequence SEQ ID NO: 1 or a variant thereof consisting of a sequence at least 85% identical to SEQ ID NO: 1; or a nucleic acid sequence encoding the polypeptide or the variant thereof, for extending the lifespan of a subject.
